# EUROPÄISCHE PATENTSCHRIFT

(11) **EP 1 221 996 B1**
(45) Veröffentlichungstag und Bekanntmachung des Hinweises auf die Patenterteilung: **22.10.2003**
(21) Anmeldenummer: 00971242.3
(22) Anmeldetag: 25.08.2000
(51) Int. Cl.: A61M 25/01

(54) **VORRICHTUNG ZUM EINFÜHREN EINES FÜHRUNGSDRAHTES UND/ODER ZUR HANDHABUNG EINES KATHETERSCHAFTES**
DEVICE FOR INSERTING A GUIDE WIRE AND/OR FOR HANDLING A CATHETER SHAFT
DISPOSITIF POUR INSERER UN FIL DE GUIDAGE ET/OU POUR MANIPULER UNE TIGE DE CATHETER

(30) Priorität: 07.10.1999 DE 19948409
(43) Veröffentlichungstag der Anmeldung: 17.07.2002
(73) Patentinhaber: Wollschläger, Helmut, 90480 Nürnberg (DE)
(72) Erfinder: Wollschläger, Helmut, 90480 Nürnberg (DE)
(74) Vertreter: Lindner, Michael
(86) Internationale Anmeldenummer: DE0002944
(87) Internationale Veröffentlichungsnummer: WO01024865

(56) Entgegenhaltungen:
- US-A- 4 716 757
- US-A- 5 159 861
- US-A- 5 318 541
- US-A- 5 427 118
- US-A- 5 443 078

## Beschreibung

Die Erfindung betrifft eine Vorrichtung zur Handhabung wenigstens eines Führungsdrahts zur Führung eines interventionellen medizinischen Instruments oder zur Handhabung eines Katheterschafts bei interventionellen medizinischen Techniken.

In der gängigen Praxis bei interventionellen medizinischen Techniken beispielsweise in der interventionellen Kardiologie oder der interventionellen Radiologie wird ein Einführventil eingesetzt, das beispielsweise bei der interventionellen Kardiologie an einen beispielsweise von der Leistengegend bis in den herznahen Bereich der Aorta gesetzten Führungskatheter angeschlossen wird. In Abhängigkeit von patientenspezifischen Erfordernissen werden in an sich bekannter Weise durch das Einführventil in den Führungskatheter hinein wenigstens ein Führungsdraht, der mit seinem distalen Ende bis in die Enden von zu behandelnden Gefäßen reicht, sowie wenigstens ein interventionelles medizinisches Instrument, beispielsweise bei der interventionellen Kardiologie eine Dilatationskatheter zur Behandlung von Stenosen, geführt. Nach Setzen des Führungsdrahts darf dieser während des gesamten Eingriffs sich in Längsrichtung nicht mehr verschieben, um zum einen Verletzungen im Endbereich der Gefäße zu vermeiden und zum anderen während der Dauer des Eingriffs einen unbehinderten Zugang zu dem Behandlungsbereich zu gewährleisten. Diese Aufgabe obliegt dem interventionellen tätigen Arzt, der durch Einklemmen des Führungsdrahts zwischen zwei Fingern dessen Längsverschiebung während der Manipulation an einem interventionellen medizinischen Instrument, beispielsweise an einem eingeführten Dilatationskatheter, bei geöffnetem Einführventil verhindert.

Diese Vorgehensweise hat sich jedoch in der interventionellen Praxis als verhältnismäßig umständlich und insbesondere im Hinblick auf auch bei sorgfältigster Vorgehensweise manchmal nicht zu vermeidenden Längsverschiebungen des Führungsdrahts für den Patienten als problematisch erwiesen.

Der Erfindung liegt die Aufgabe zugrunde, eine Vorrichtung zur Handhabung wenigstens eines Führungsdrahts zur Führung eines interventionellen medizinischen Instruments oder zur Handhabung eines Katheterschafts bei interventionellen medizinischen Techniken zu schaffen, die dem interventionell tätigen Arzt eine vereinfachte Manipulation der interventionellen Instrumente bei einem Eingriff gestattet.

Diese Aufgabe wird erfindungsgemäß gelöst mit einer Vorrichtung zur Handhabung wenigstens eines Führungsdrahts zur Führung eines interventionellen medizinischen Instruments oder eines Katheterschafts bei interventionellen medizinischen Techniken mit einem an einem Einführventil befestigbaren Fixierarm und mit einer an dem Fixierarm angebrachten Klemmvorrichtung, mit der der betreffende Führungsdraht beziehungsweise Katheterschaft gegen Längsverschiebung gesichert fixierbar ist.

Dadurch, daß ein Fixierarm mit einer an dem Fixierarm angebrachten Klemmvorrichtung zur Fixierung eines Führungsdrahts beziehungsweise Katheterschaft gegen Längsverschiebungen vorgesehen ist, wird der interventionell tätige Arzt nach Setzen eines Führungsdrahts oder nach Legen eines wenigstens zeitweise zu fixierenden interventionellen medizinischen Instruments wie beispielsweise eines Katheters während des Eingriffs von der Fixierung des Führungsdrahts beziehungsweise eines dem gesetzten interventionellen medizinischen Instrument zugeordneten Katheterschafts entlastet und kann sich mit voller Aufmerksamkeit anderen Arbeiten wie beispielsweise der Betätigung des Einführventils mit einer Hand und der Manipulation eines interventionellen medizinischen Instruments mit der anderen Hand widmen. Dadurch sind die Risiken für einen Patienten bei interventionellen medizinischen Techniken erheblich gemindert.

Bei einer zweckmäßigen Weiterbildung der erfindungsgemäßen Vorrichtung ist vorgesehen, daß der Fixierarm über eine Nut-Feder-Verbindung lösbar an dem Einführventil befestigbar ist. Bei einer weiteren zweckmäßigen Weiterbildung der erfindungsgemäßen Vorrichtung ist vorgesehen, daß der Fixierarm über eine Steckverbindung lösbar an dem Einführventil befestigbar ist. Bei der letztgenannten Weiterbildung ist in einer Ausgestaltung ein Adapterstück vorgesehen, mit dem der Fixierarm an dem Einführventil lösbar befestigbar ist. Dadurch ist jeweils eine verhältnismäßig einfache Anbringung des Fixierarms an dem Einführventil erzielt.

Bei einer weiteren zweckmäßigen Weiterbildung ist vorgesehen, daß der Fixierarm fest mit dem Einführventil verbunden ist. Bei dieser Weiterbildung ist sichergestellt, daß der Fixierarm während des Eingriffs sich unter keinen Umständen von dem Einführventil lösen kann.

Im Hinblick auf eine einfache Fixierbarkeit ist zweckmäßigerweise vorgesehen, daß der Fixierarm über ein Einführende des Einführventils vorsteht. Dadurch sind die zu fixierenden Elemente unter Vermeidung von starken Biegungen verhältnismäßig geradlinig gehalten.

Bei der letztgenannten Ausgestaltung ist weiterhin zweckmäßig, daß der Fixierarm einen Befestigungsabschnitt und einen gegen den Befestigungsabschnitt abgewinkelten Halteabschnitt aufweist. Dadurch bleibt der dem Einführende des Einführventils benachbarte Bereich verhältnismäßig ungehindert zugänglich.

Um eine Klemmvorrichtung mit mehreren Klemmeinheiten verwenden zu können, ist es zweckmäßig, daß der Fixierarm einen Befestigungsabschnitt und einen als Halteplatte ausgebildeten Halteabschnitt aufweist.

Im Hinblick auf eine möglichst kompakte Ausgestaltung ist es zweckmäßig, daß die Klemmvorrichtung eine Klemmeinheit zur Fixierung eines Führungsdrahts aufweist.

Sollen mehrere Elemente fixiert werden, ist es zweckmäßig, daß die Klemmvorrichtung wenigstens zwei jeweils in einem seitlichen Abstand angeordnete Klemmeinheiten zur Fixierung von zwei Führungsdrähten oder einem Führungsdraht und einem Katheterschaft aufweist. In diesem Zusammenhang ist es insbesondere vorteilhaft, daß die Klemmvorrichtung drei oder vier jeweils in einem seitlichen Abstand angeordnete Klemmeinheiten zur Fixierung von Führungsdrähten und/oder Katheterschäften aufweist.

Bei einer Weiterbildung ist vorgesehen, daß die Klemmvorrichtung als Klemmeinheit wenigstens eine längliche Klemmschiene aufweist, die über ein Fassungsteil mit einer Grundplatte sowie zwei Seitenwangen und über einen in das Fassungsteil eingefügten, mit einem längs verlaufenden Klemmschlitz ausgebildeten Klemmkörper verfügt. Dadurch werden insbesondere auf einen in der Regel verhältnismäßig glatten Führungsdraht ausreichend hohe Reibungskräfte ausgeübt.

Bei einer Ausgestaltung der letztgenannten Weiterbildung ist es im Hinblick auf eine mechanisch einfache Bauausführung zweckmäßig, daß die Grundplatte sowie die Seitenwangen fest miteinander verbunden sind und rechtwinklig zueinander stehen.

Insbesondere bei verhältnismäßig dicken Querschnitten von durch Klemmschienen gemäß der letztgenannten Weiterbildung zu fixierenden Elementen ist es gemäß einer weiteren Ausgestaltung zweckmäßig, daß die Seitenwangen jeweils über ein Scharnier schwenkbar an der Grundplatte angebracht sind und daß eine Verriegelungsvorrichtung zur Fixierung der bei geschlossenem Klemmschlitz vorgesehen ist.

Bei einer zweckmäßigen Weiterbildung der Verriegelungsvorrichtung ist vorgesehen, daß die Verriegelungsvorrichtung zwei Schnappnasen, die sich in geschlossener Stellung der Seitenwangen gegenseitig hintergreifen.

Bei einer weiteren zweckmäßigen Weiterbildung der Verriegelungsvorrichtung ist vorgesehen, daß die Verriegelungsvorrichtung einen Kippbügel aufweist, der mit einem Ende gelenkig an einer Seitenwange angebracht ist und in geschlossener Stellung die andere Seitenwange übergreift.

Zur Erhöhung der Reibungskräfte in einem Klemmschlitz ist zweckmäßigerweise vorgesehen, daß der Klemmschlitz gewellt ist.

Weiterhin ist in Weiterbildungen zur Erhöhung der Reibungskräfte in einem Klemmschlitz vorgesehen, daß der Klemmkörper zu dem Klemmschlitz quer oder schräg ausgerichtete Schlitze aufweist.

Bei einer weiteren Ausgestaltung einer Klemmvorrichtung ist vorgesehen, daß die Klemmvorrichtung wenigstens eine Klemmhülsenanordnung mit einer Klemmhülse und einer Schraubhülse aufweist, die mit zueinander ausrichtbaren, zur Aufnahme eines Führungsdrahts oder eines Katheterschafts vorgesehene Nuten ausgebildet sind, wobei sich in einer Drehrichtung der Querschnitt der Klemmhülse wenigstens abschnittsweise verringert.

Bei dieser Ausgestaltung das in sich vorteilhafterweise die auf die eingeführten Elemente ausgeübten Reibungskräfte einstellen.

Bei einer weiteren Ausgestaltung einer Klemmvorrichtung ist vorgesehen, daß die Klemmvorrichtung eine Gegenplatte und einen drehbar gelagerten Schwenkkörper aufweist, der in einer ersten Schwenkstellung einen Abstand von der Gegenplatte aufweist und in einer zweiten Schwenkstellung abschnittsweise an der Gegenplatte anliegt.

Bei einer diesbezüglichen Weiterbildung ist vorgesehen, daß der Schwenkkörper einen kreisartigen Querschnitt aufweist und azentrisch gelagert ist.

Bei einer anderen diesbezüglichen Weiterbildung ist vorgesehen, daß der Schwenkkörper einen ellipsenartigen Querschnitt aufweist. Bei der Weiterbildung mit einem Schwenkkörper mit einem ellipsenartigen Querschnitt ist vorgesehen, daß der Schwenkkörper mittig gelagert ist. Bei einer anderen Weiterbildung mit einem Schwenkkörper mit einem ellipsenartigen Querschnitt ist vorgesehen, daß der Schwenkkörper im Bereich eines Brennpunkts gelagert ist.

Die Ausgestaltung und diesbezüglichen Weiterbildungen mit einem Schwenkkörper zeichnen sich aufgrund der Ausnutzung der Hebelwirkung durch eine besonders zuverlässige Fixierung aus.

Weitere zweckmäßige Ausgestaltungen der Erfindung sind Gegenstand der Unteransprüche sowie der nachfolgenden Beschreibung von Ausführungsbeispielen der Erfindung unter Bezug auf die Figuren der Zeichnung. Es zeigen:
- Fig. 1: in einer Seitenansicht ein Einführventil mit einem daran angebrachten Fixierarm, an dem eine Klemmschiene angebracht ist,
- Fig. 2: in einer Seitenansicht ein Einführventil mit einem daran angebrachten Fixierarm, an dem drei Klemmschienen angebracht sind,
- Fig. 3: in einer Rückansicht ein Einführventil gemäß Fig. 1 beziehungsweise Fig. 2,
- Fig. 4: in einer Seitenansicht ein Einführventil gemäß Fig. 1 beziehungsweise Fig. 2 bei abgenommenem Fixierarm,
- Fig. 5 und Fig. 6: in einer Draufsicht beziehungsweise in einer Stirnansicht eine einen Klemmkörper aufweisenden Klemmschiene gemäß Fig. 1 beziehungsweise Fig. 2,
- Fig. 7 und Fig. 8: jeweils in einer Draufsicht weitere Ausgestaltungen von Klemmkörper für Klemmschienen gemäß Fig. 1 und Fig. 2,
- Fig. 9: in einem Schnitt eine Ausgestaltung einer Klemmschiene mit klappbaren Seitenwangen,
- Fig. 10: in einem Schnitt eine weitere Ausgestaltung einer Klemmschiene mit klappbaren Seitenwangen,
- Fig. 11: in einer Seitenansicht ein Einführventil mit einem Fixierarm, an dem eine Klemmhülsenanordnung angebracht ist,
- Fig. 12: in einem Schnitt die Klemmhülsenanordnung gemäß Fig. 11 in einer Einlegestellung,
- Fig. 13 und Fig. 14: in einem Schnitt beziehungsweise einer Ansicht die Klemmhülsenanordnung gemäß Fig. 11 in einer Fixierstellung,
- Fig. 15 und Fig. 16: in einer Draufsicht eine Schwenkkörperanordnung in einer Einlegestellung beziehungsweise in einer Fixierstellung.

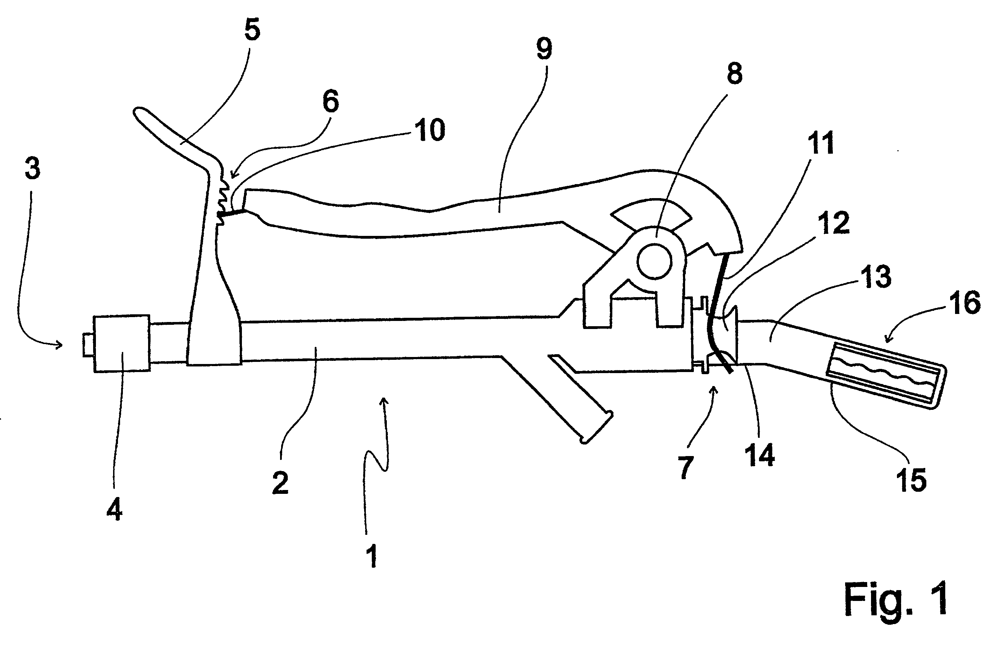

Fig. 1 zeigt in einer Seitenansicht ein in Verbindung mit der Erfindung aufgrund der einhändigen Bedienbarkeit besonders vorteilhaft verwendetes Einführventil 1, das über einen länglichen Ventilkörper 2 verfügt. An einem Anschlußende 3 ist der Ventilkörper 2 mit einer Schraubmanschette 4 ausgestattet, die auf einen in Fig. 1 nicht dargestellten Führungskatheter aufschraubbar ist. Im Bereich des Anschlußendes 3 ist an dem Ventilkörper 2 ein biegsamer Rastarm 5 angebracht, der über eine Anzahl von Rastnasen 6 verfügt. Weiterhin ist im Bereich eines dem Anschlußende 3 gegenüberliegenden Einführendes 7 ein an dem Ventilkörper 2 befestigtes Lagerstück 8 vorgesehen, an dem ein Handgriff 9 drehbar gelagert ist. An dem dem Lagerstück 8 abgewandten Ende des Handgriffs 9 ist eine Rastzunge 10 angebracht, die bei einer in etwa parallelen Ausrichtung des Handgriffs 9 zu dem Ventilkörper 2 mit den Rastnasen 6 in Eingriff kommt.

Mit dem Handgriff 9 ist eine Zunge 11 aus Metall oder Kunststoff betätigbar, die mit einem an dem Einführende 7 in den Ventilkörper 2 eingeführten Druckkolben 12 in Eingriff steht. In der in Fig. 1 dargestellten Einführstellung des Handgriffs 9 ist eine Einführöffnung freigegeben, durch die beispielsweise ein Führungsdraht für im Bereich von interventionellen medizinischen Techniken verwendete Interventionsinstrumente, beispielsweise Interventionskatheter wie Dilatationskatheter in der interventionellen Kardiologie oder entsprechende Instrumente in der interventionellen Radiologie beziehungsweise Bohrer oder Fräsen, sowie nach Legen des Führungskatheters das betreffende Interventionsinstrument selbst einführbar ist.

Schließlich mündet in etwa im Bereich des Lagerstücks 8 in spitzwinkliger Ausrichtung ein Seitenrohr in den Ventilkörper 2, an dem in an sich bekannter Weise zur Überwachung der Druckverhältnisse ein Manometer anschließbar ist.

Es versteht sich, daß auch andersartig ausgestaltete Einführventile, die beispielsweise mit einem Drehverschluß zur Abdichtung des Einführendes 7 ausgestattet sind, in Verbindung mit der Erfindung verwendbar sind.

Bei dem Ausführungsbeispiel gemäß Fig. 1 ist an dem Ventilkörper 2 des Einführventils 1 ein Fixierarm 13 angebracht, der über einen mit der Längsachse des Ventilkörpers 2 ausgerichteten, mit dem Ventilkörper 2 verbundenen Befestigungsabschnitt 14 und sowie über einen gegenüber dem Befestigungsabschnitt 14 abgewinkelten Halteabschnitt 15 verfügt. An dem Halteabschnitt 15 ist als Klemmvorrichtung für einen Führungsdraht eine längliche Klemmschiene 16 als Klemmeinheit angebracht, mit dem der Führungsdraht oder auch ein Katheterschaft eines Interventionsinstruments gegen Längsverschiebungen fixierbar ist.

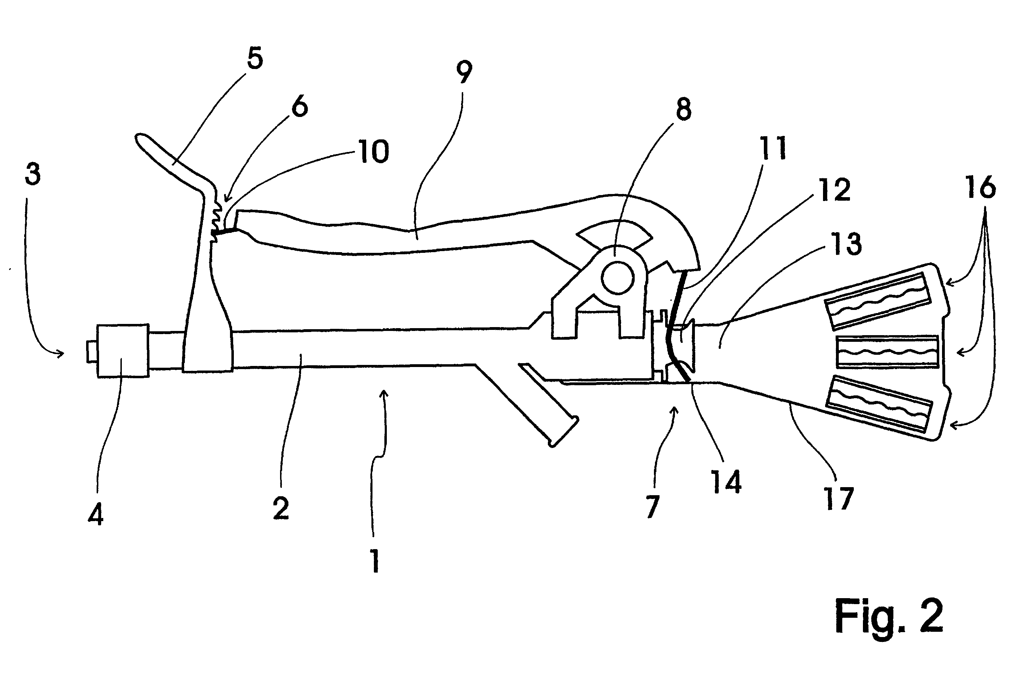

Fig. 2 zeigt in einer Seitenansicht ein Einführventil 1 gemäß Fig. 1 mit einem Fixierarm 13, an dem an einer sich an den Befestigungsabschnitt 14 anschließenden Halteplatte 17 drei Klemmschienen 16 angebracht sind. Mit den drei Klemmschienen 16 sind beispielsweise zwei Führungsdrähte und ein Führungsabschnitt eines Interventionsinstruments oder ein Führungsdraht und Führungsabschnitte von zwei Interventionsinstrumenten gegen Längsverschiebungen fixierbar.

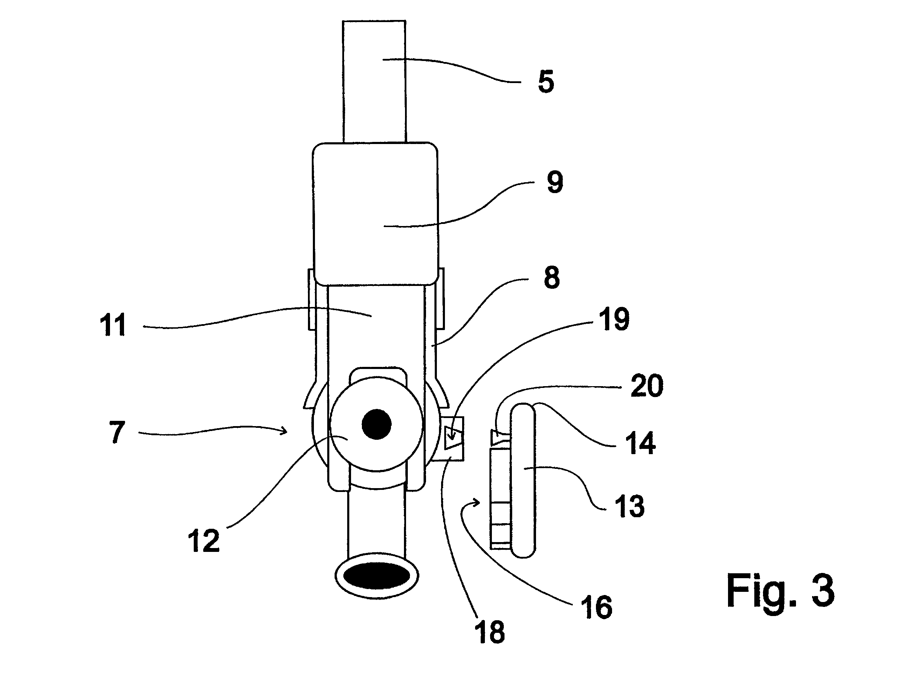

Fig. 3 zeigt in einer Rückansicht auf das Einführende 7 ein Einführventil 1 gemäß Fig. 1 beziehungsweise Fig. 2 sowie den Fixierarm 13 gemäß Fig. 1. Aus Fig. 3 ist ersichtlich, daß an dem Ventilkörper 2 eine Steckschiene 18 ausgebildet ist, die über eine sich zu ihrer Öffnung verjüngende Nut 19 verfügt. An dem Befestigungsabschnitt 14 des Fixierarms 13 ist ein Steg 20 vorgesehen, der eine zu dem Querschnitt der Nut 19 komplementäre Form aufweist. Somit sind das Einführventil 1 und der Fixierarm 13 nach Art einer Nut-Feder-Verbindung lösbar miteinander verbindbar.

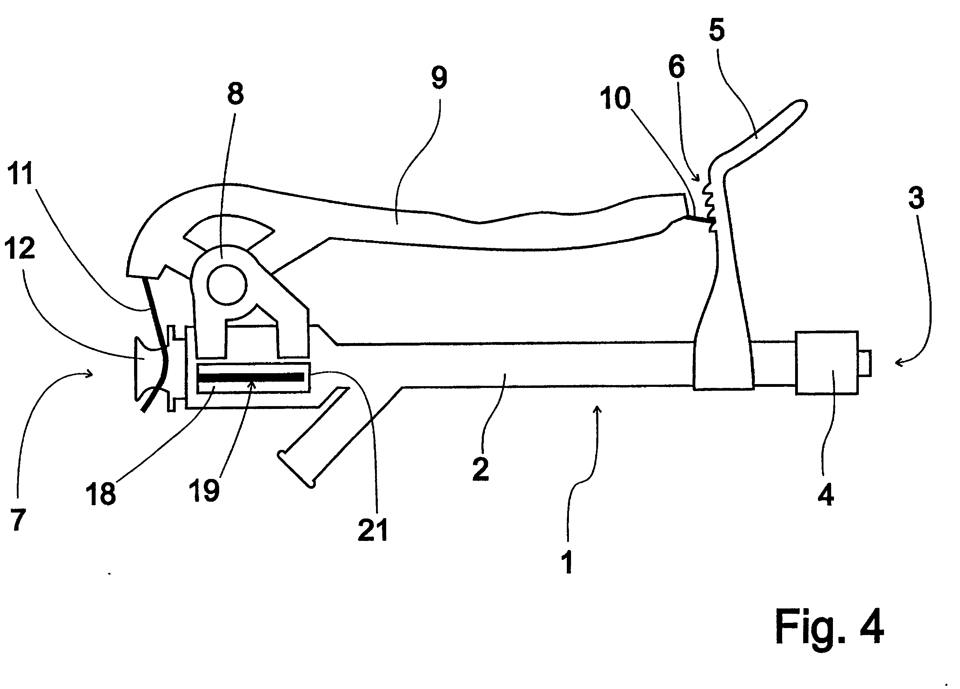

Fig. 4 zeigt in einer Seitenansicht ein Einführventil 1 gemäß Fig. 1 beziehungsweise Fig. 2 bei abgenommenem Fixierarm 13. Aus Fig. 4 ist ersichtlich, daß die Steckschiene 18 eine Länge aufweist, die zu einer spielfreien Halterung des Fixierarms 13 führt. An dem von dem Einführende 7 wegweisenden Ende ist die Nut 19 der Steckschiene 18 mit einer Abschlußwand 21 abgeschlossen, an die der Steg 20 des Tragarms 13 nach im wesentlichen vollständigen Einführen in die Nut 19 anschlägt, so daß ein unbeabsichtigtes Durchschieben verhindert ist.

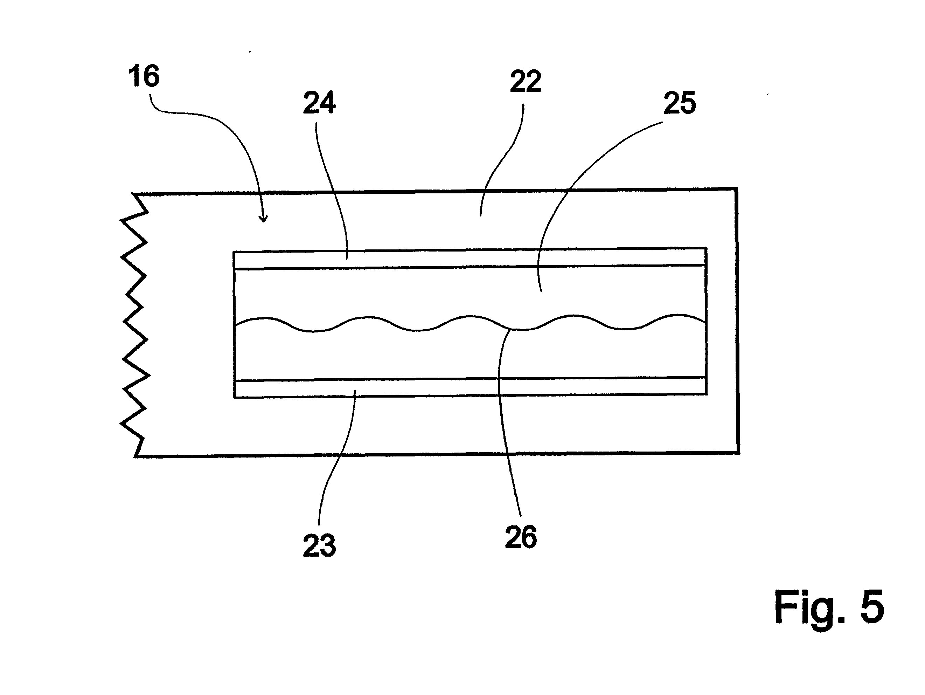

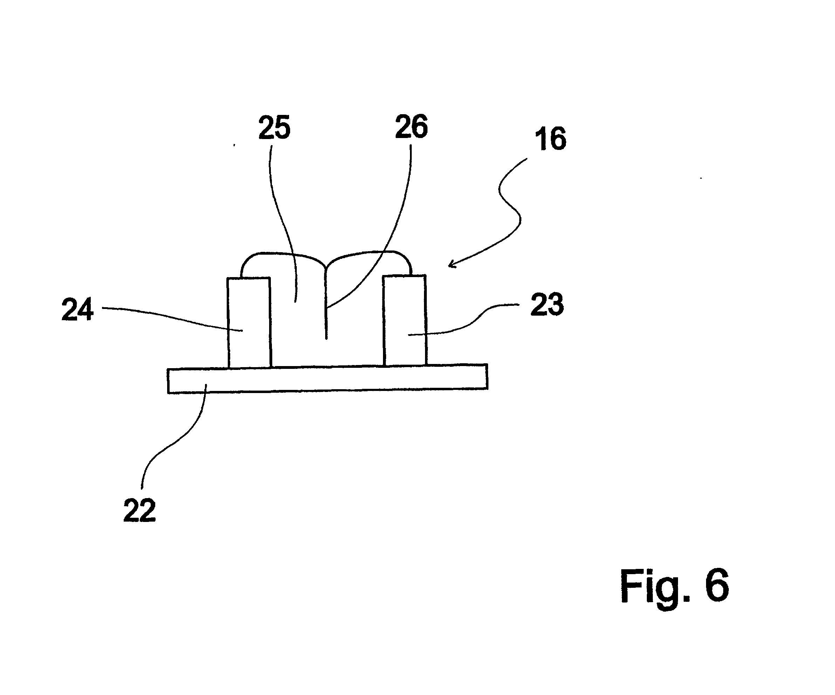

Fig. 5 und Fig. 6 zeigen in einer Draufsicht beziehungsweise in einer Stimansicht eine Klemmschiene 16 gemäß Fig. 1 beziehungsweise Fig. 2. Die Klemmschiene 16 weist als Fassungsteil eine durch den Halteabschnitt 15 beziehungsweise die Halteplatte 17 des Fixierarms 13 gebildete Grundplatte 22 auf, an der in rechtwinkliger Ausrichtung eine längliche erste Seitenwange 23 und eine längliche zweite Seitenwange 24 angebracht sind. Zwischen den Seitenwangen 23, 24 ist ein Klemmkörper 25 eingebracht, der aus einem verhältnismäßig weichen, komprimierbaren Material wie beispielsweise Silikon mit einem hohen Haftreibungskoeffizienten gefertigt ist. in etwa mittig ist in Längsrichtung ein Klemmschlitz 26 in den Klemmkörper 25 eingebracht, in den beispielsweise ein Führungsdraht einfügbar ist. Der Klemmschlitz 26 in dem Klemmkörper 25 ist vorteilhafterweise wie in Fig. 5 dargestellt gewellt ausgeführt, um die auf einen eingefügten, verhältnismäßig glatten und steifen Führungsdraht ausgeübten Reibungskräfte zu erhöhen.

Aus Fig. 6 ist ersichtlich, daß der Klemmschlitz 26 vorteilhafterweise in einem Abstand von der Grundplatte 22 endet, um den Klemmkörper 25 für eine vereinfachte Handhabung einstückig zu halten.

Es versteht sich, daß bei Abwandlungen der Klemmschlitz 26 auch durchgehend und der Klemmkörper 25 zweiteilig ausgebildet sein kann.

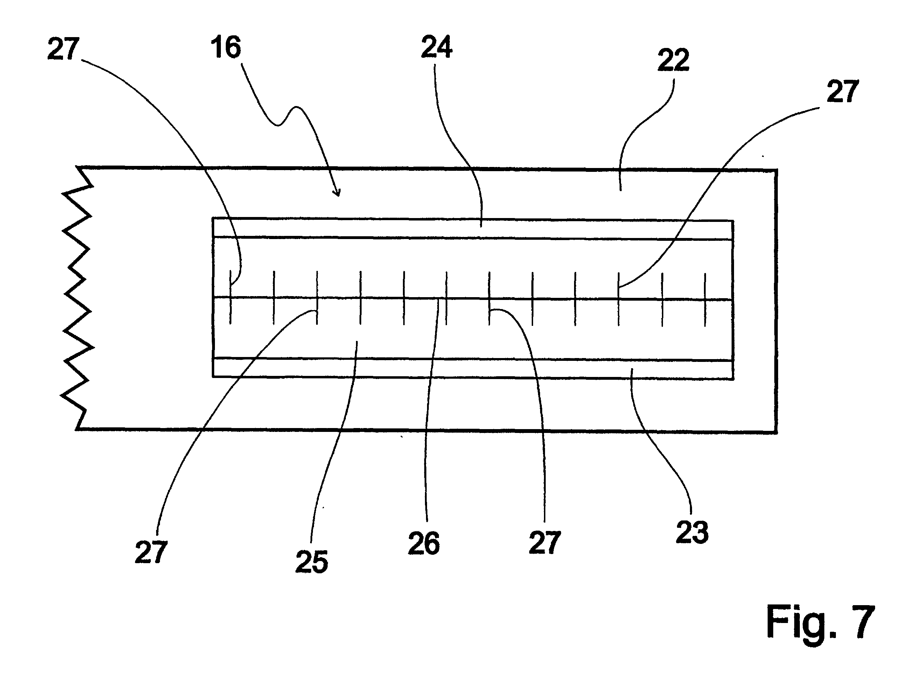

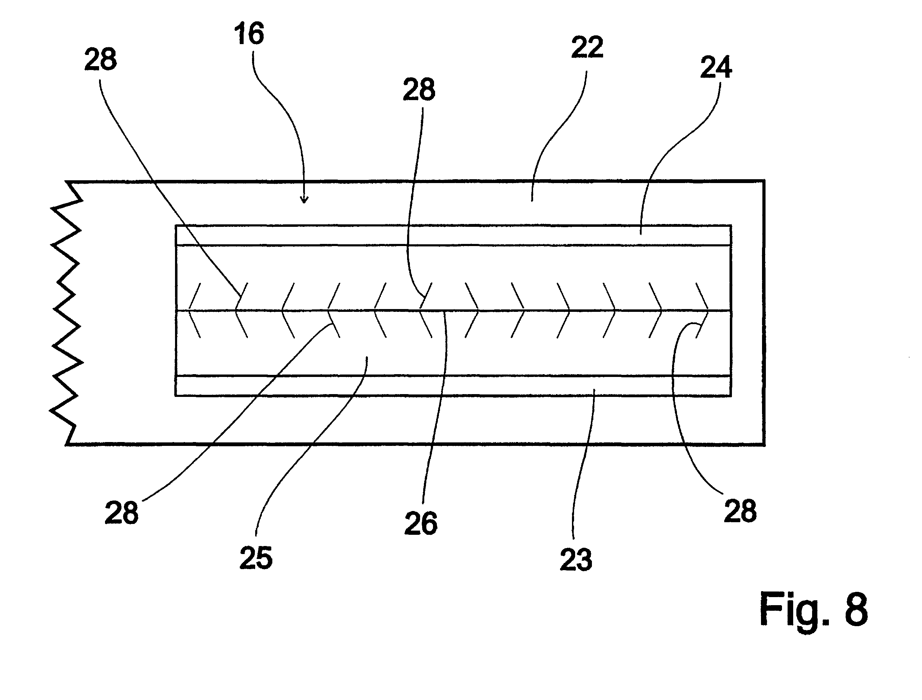

Fig. 7 und Fig. 8 zeigen in einer Draufsicht weitere Ausgestaltungen von Klemmkörper 25 für Klemmschienen 16 gemäß Fig. 1 und Fig. 2. Bei der Ausgestaltung gemäß Fig. 7 ist der Klemmschlitz 26 in Längsrichtung der Klemmschiene 16 gerade ausgeführt. Zur Erhöhung der Reibungskräfte sind rechtwinklig zu dem Klemmschlitz 26 eine Anzahl von Querschlitze 27 in den Klemmkörper 25 eingebracht, die sich bei Ausübung einer Zugkraft auf einen eingeführten Führungsdraht oder Katheterschaft verkeilen. Bei der Ausgestaltung gemäß Fig. 8 ist der Klemmschlitz 26 in Längsrichtung der Klemmschiene 16 ebenfalls gerade ausgeführt, wobei jedoch in Abwandlung zu der Ausgestaltung gemäß Fig. 7 eine Anzahl von schräg zu dem Klemmschlitz 26 ausgerichtete Schrägschlitze 28 in den Klemmkörper 25 eingebracht sind. Die Schrägschlitze 28 sind in zwei Gruppen mit jeweils gegensinniger Ausrichtung angeordnet, um bei Ausübung von Zugkräften in beide Richtungen die Reibungskräfte durch Verkeilung zu erhöhen.

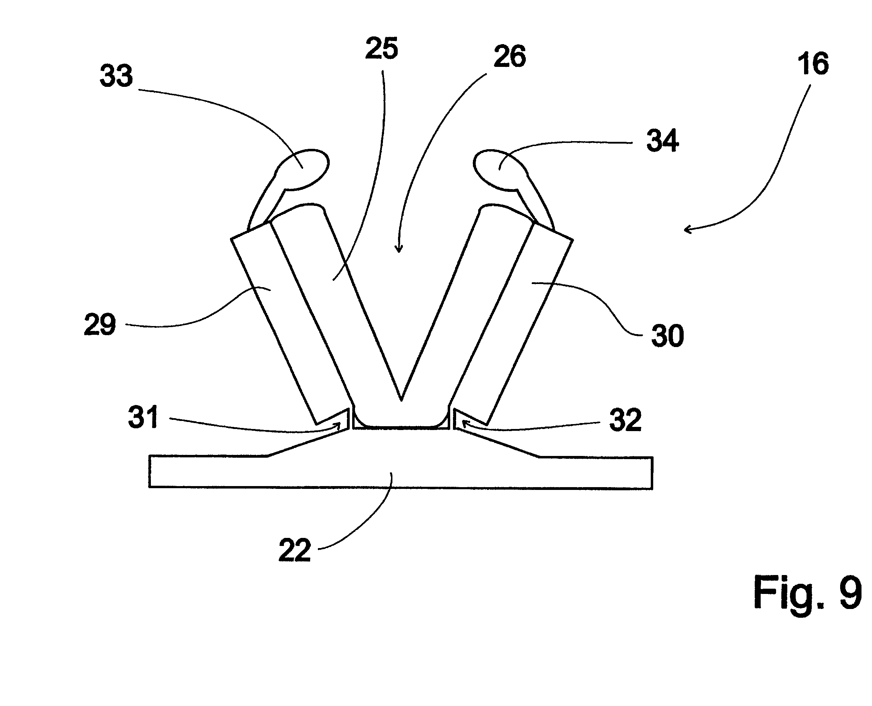

Fig. 9 zeigt in einem Schnitt eine Ausgestaltung einer Klemmschiene 16, die entsprechend den in Fig. 1 bis Fig. 3, Fig. 5 bis Fig. 8 dargestellten Klemmschienen 16 über eine Grundplatte 22 sowie einen Klemmkörper 25 verfügt, in den ein Klemmschlitz 26 eingebracht ist. Die Klemmschiene 16 gemäß Fig. 9 ist mit einer klappbaren ersten Seitenwange 29 sowie einer klappbaren zweiten Seitenwange 30 ausgebildet, die über ein erstes Scharnier 31 beziehungsweise über ein zweites Scharnier 32 mit der Grundplatte 22 verbunden sind. Bei der Ausgestaltung gemäß Fig. 9 sind die Scharniere 31, 32 als gegenüber der Dicke der klappbaren Seitenwangen 29, 30 verhältnismäßig dünn ausgebildete Stege ausgeführt, die eine zum Klappen der Seitenwangen 29, 30 ausreichende Elastizität sowie eine für wenigstens eine Anzahl von einigen Dutzend Klappvorgängen ausreichende Bruchsicherheit aufweisen.

An den von den Scharnieren 31, 32 wegweisenden Enden der klappbaren Seitenwangen 29, 30 ist als Verriegelungsvorrichtung ein aus einer ersten Schnappnase 33 und einer zweiten Schnappnase 34 gebildeter Schnappverschluß vorgesehen, bei dem sich in geschlossener Stellung mit einer im wesentlichen parallelen Ausrichtung der klappbaren Seitenwangen 29, 30 die Schnappnasen 33, 34 gegenseitig hintergreifen. Zum Öffnen der klappbaren Seitenwangen 29, 30 ist der Schnappverschluß dadurch öffenbar, daß die im Endbereich jeweils rundlich verdickt ausgebildeten Schnappnasen 33, 34 von Hand seitlich aneinander vorbeigeschoben werden.

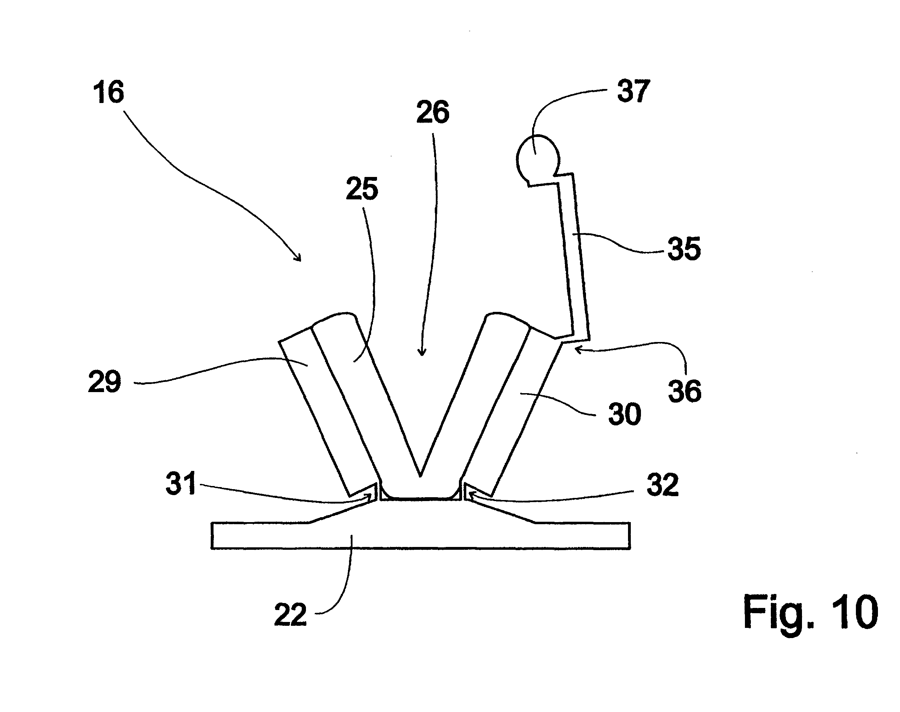

Fig. 10 zeigt in einem Schnitt eine weitere Ausgestaltung einer Klemmschiene 16 mit klappbaren Seitenwangen 29, 30 entsprechend der Ausgestaltung gemäß Fig. 9, bei der als Verriegelungsvorrichtung in Abweichung von der Ausgestaltung gemäß Fig. 9 ein Kippbügel 35 vorgesehen ist. Der Kippbügel 35 ist über ein in der Ausgestaltung gemäß Fig. 10 als Steg ausgebildetes Bügelschamier 36 mit dem von dem betreffenden Scharnier 31, 32 wegweisenden Ende einer klappbaren Seitenwange 29, 30 verbunden und an der dem Bügelschamier 36 gegenüberliegenden Ende rechtwinklig abgewinkelt sowie zur Erleichterung der Handhabung im Bereich des abgewinkelten Abschnitts mit einem Betätigungsknopf 37 ausgebildet. Zum Schließen des Klemmschlitzes 26 werden die klappbaren Seitenwangen 29, 30 aufeinander zu bewegt, und bei einer im wesentlichen parallelen Ausrichtung derselben wird der Kippbügel 35 so umgelegt, daß der abgewinkelte Abschnitt die dem Bügelschamier 36 gegenüberliegende Seitenwange 29, 30 hintergreift.

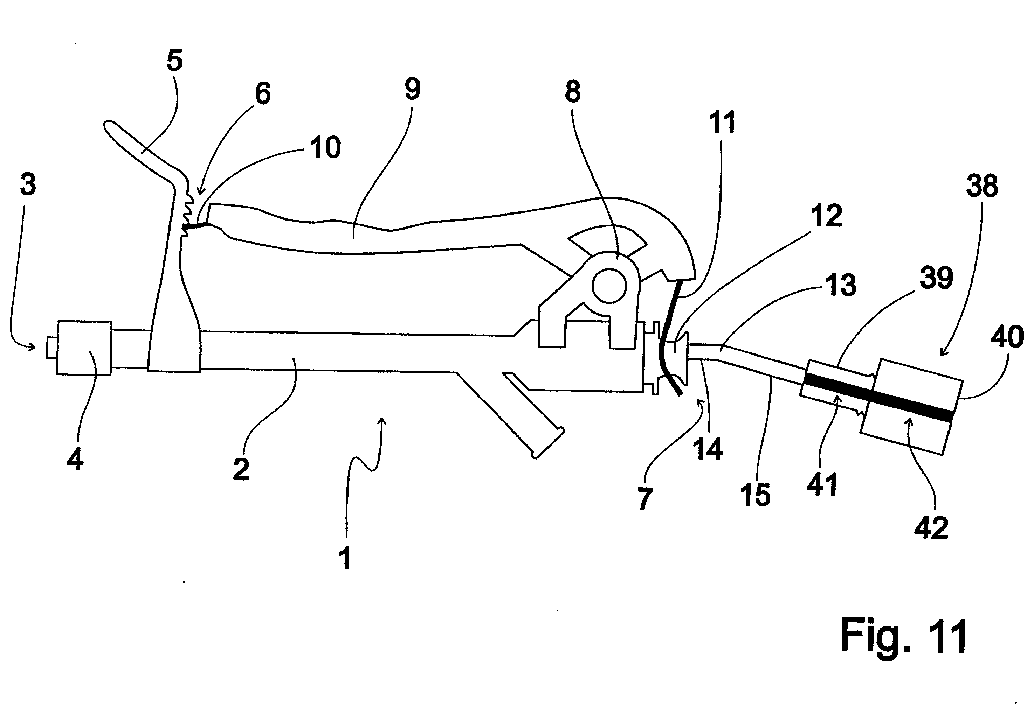

Fig. 11 zeigt in einer Seitenansicht ein entsprechend den in Fig. 1 bis Fig. 4 dargestellten Ausführungsbeispielen ausgebildetes Einführventil 1 mit einem Fixierarm 13, an dem eine Klemmhülsenanordnung 38 als Klemmeinheit einer Klemmvorrichtung angebracht ist. Die Klemmhülsenanordnung 38 weist eine mit dem Halteabschnitt 15 des Fixierarms 13 drehfest verbundene Klemmhülse 39 sowie eine gegenüber der Klemmhülse 39 verdrehbare Schraubhülse 40 auf. Die Klemmhülse 39 verfügt über eine Klemmhülsennut 41, die in der in Fig. 11 dargestellten Einlegestellung mit einer in der Schraubhülse 40 ausgebildeten Schraubhülsennut 42 ausgerichtet ist, so daß in die Klemmhülsenanordnung 38 beispielsweise ein Führungsdraht einlegbar ist.

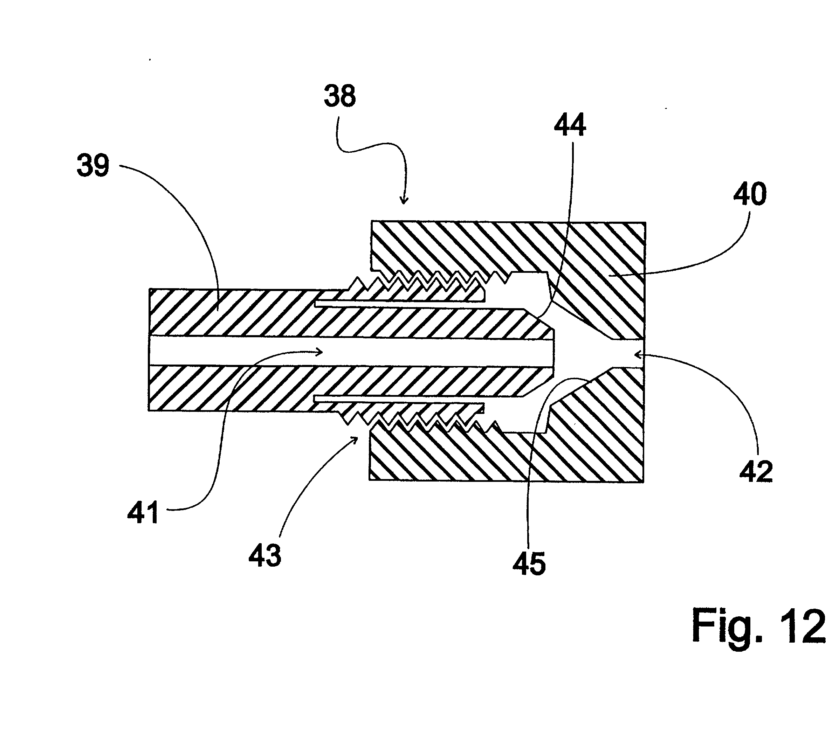

Fig. 12 zeigt in einem Schnitt die Klemmhülsenanordnung 38 gemäß Fig. 11 in der Einlegestellung. Aus Fig. 12 ist ersichtlich, daß die Klemmhülse 39 und die Schraubhülse 40 über ein Gewinde 43 drehbar miteinander verbunden sind, wobei die Klemmhülse 39 abschnittsweise in die Schraubhülse 40 eingreift. An ihren jeweils aufeinander zu weisenden Endseiten sind die Klemmhülse 39 und die Schraubhülse 40 mit zueinander komplementären Abschrägungen 44, 45 ausgebildet, die in der Einlegestellung voneinander beabstandet sind beziehungsweise ohne wesentliche Deformation des in die Schraubhülse 40 eingreifenden Endes der Klemmhülse 39 aneinander anliegen.

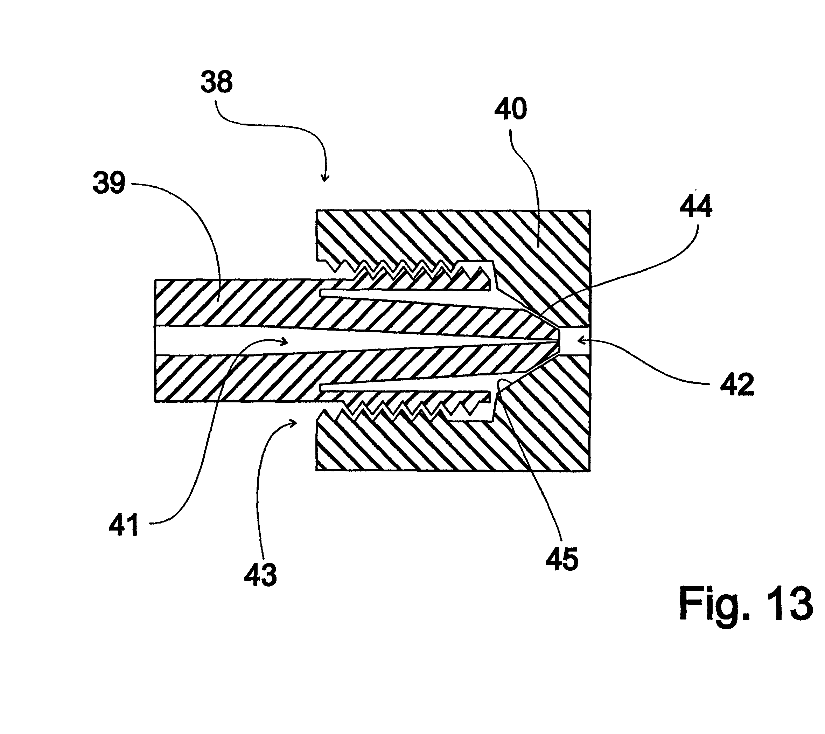

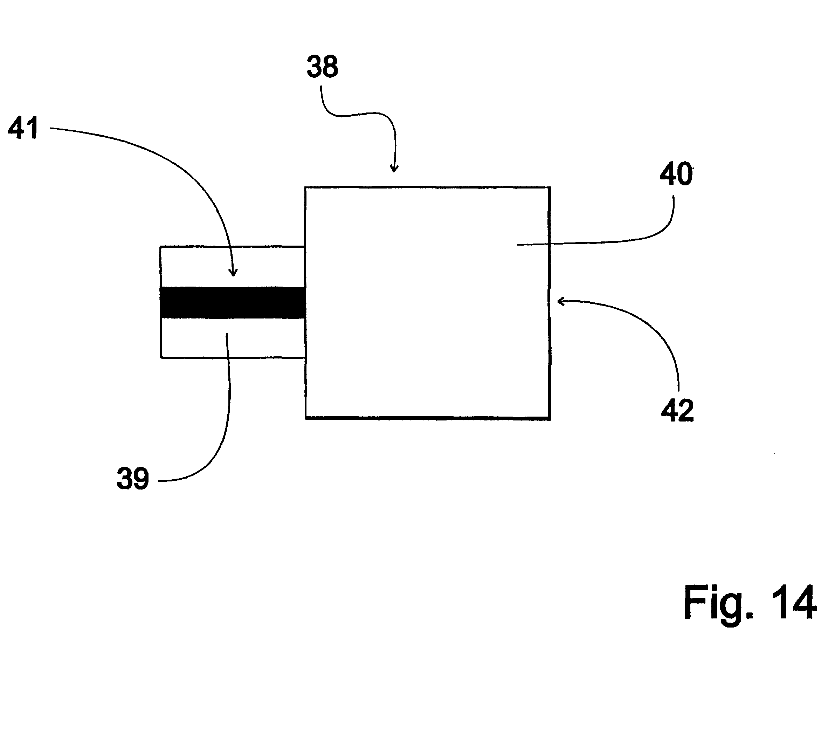

Fig. 13 und Fig. 14 zeigen in einem Schnitt beziehungsweise einer Ansicht die Klemmhülsenanordnung 38 gemäß Fig. 11 in einer Fixierstellung, in der die Schraubhülse 40 so weit auf die Klemmhülse 39 aufgeschraubt ist, daß die Abschrägungen 44, 45 der Klemmhülse 39 beziehungsweise der Schraubhülse 40 miteinander in Eingriff kommen und unter Verengung der Klemmhülsennut 41 im endseitigen Bereich aneinander vorbeigleiten.

Aus Fig. 14 ist ersichtlich, daß in der Fixierstellung die Klemmhülsennut 41 und die in der Darstellung gemäß Fig. 14 nicht sichtbare Schraubhülsennut 42 gegeneinander verdreht sind.

In der Fixierstellung der Klemmhülsenanordnung 38 ist somit ein in die Klemmhülsennut 41 und die Schraubhülsennut 42 eingelegter Führungsdraht oder Katheterschaft gegen Längsverschiebung gesichert. Zur Entnahme des Führungsdrahts oder Katheterschafts ist die Schraubhülse 40 gegen die Klemmhülse 39 soweit zu verdrehen, daß die Klemmhülsennut 41 wieder ausreichend geöffnet und mit der Schraubhülsennut 42 ausgerichtet ist.

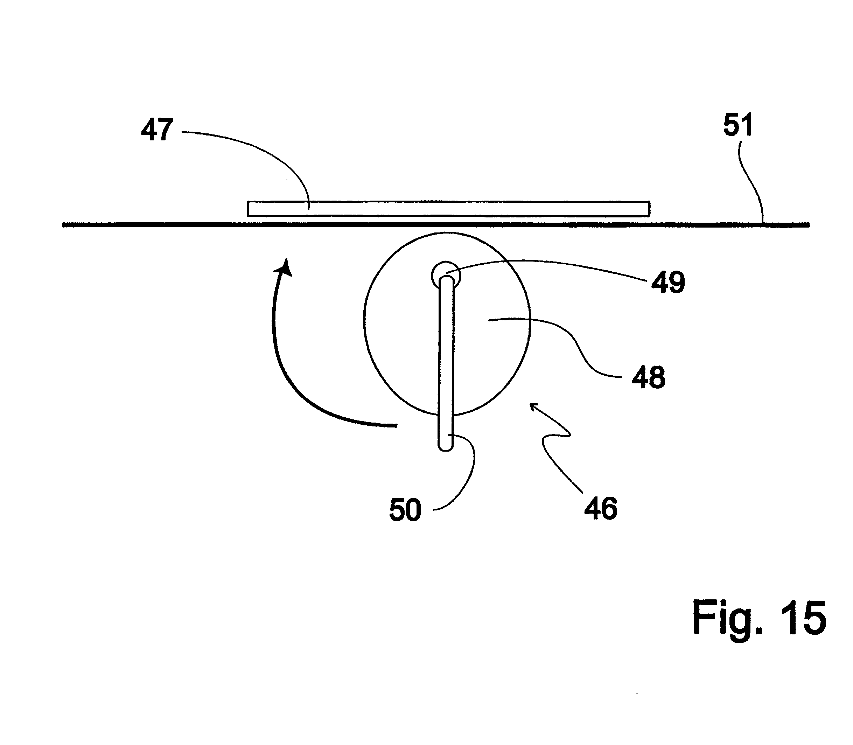

Fig. 15 zeigt in einer Draufsicht eine mit einem in Fig. 15 nicht dargestellten Einführventil 1 beispielsweise gemäß Fig. 1 bis Fig. 4 verbindbare Schwenkkörperanordnung 46 in einer Einlegestellung. Die Schwenkkörperanordnung 46 weist eine Gegenplatte 47 auf, die mit einem in Fig. 15 nicht dargestellten Fixierarm 13 zur Befestigung an einem Einführventil 1 verbunden oder einstückig mit diesem ausgebildet ist. Weiterhin ist die Schwenkkörperanordnung 46 mit einem komprimierbaren Schwenkkörper 48 ausgestattet, der um eine Achse 49 schwenkbar ist. Die Achse 49 ist in einem Abstand von der Gegenplatte 47 angeordnet, der größer als die geringste Materialdicke zwischen der Achse 49 und der Außenseite des Schwenkkörpers 48 ist. Zur Betätigung des Schwenkkörpers 48 ist ein Hebel 50 vorgesehen, der mit dem Schwenkkörper 48 verbunden ist.

Bei dem in Fig. 15 dargestellten Ausführungsbeispiel ist der Schwenkkörper 48 ellipsenartig ausgeführt und die Achse 49 azentrisch beispielsweise im Bereich eines Brennpunkts angeordnet. In der in Fig. 15 dargestellten Einlegestellung ist bei entsprechender Ausrichtung des Hebels 50 zwischen der Gegenplatte 47 und der der Gegenplatte 47 zugewandten Seite des Schwenkkörpers 48 ein Zwischenraum vorhanden, in den beispielsweise ein Führungsdraht 51 einführbar ist.

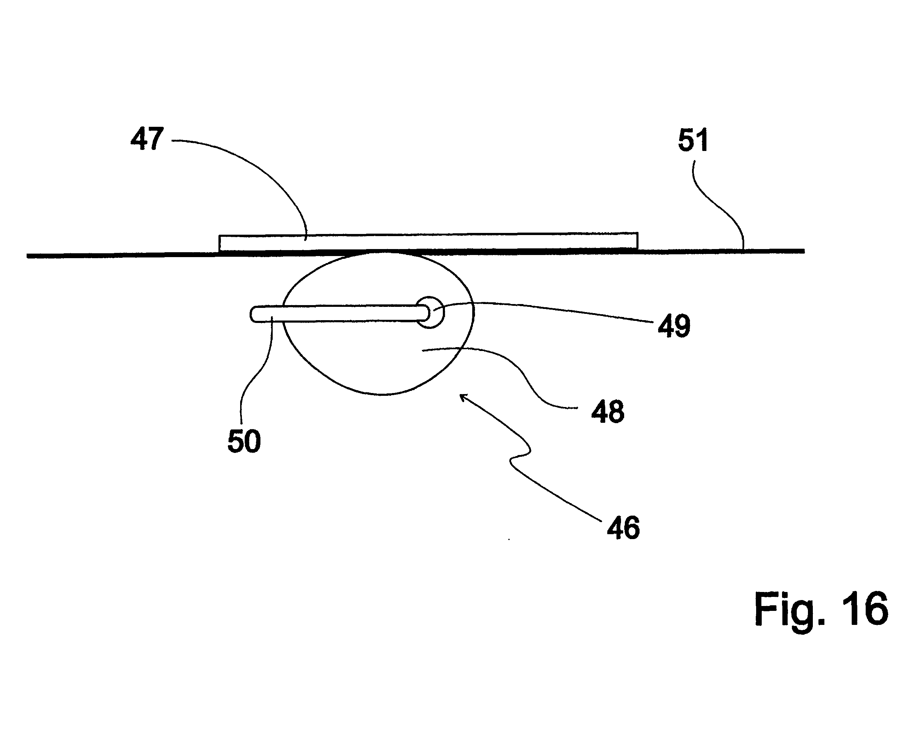

Fig. 16 zeigt die Schwenkkörperanordnung 46 gemäß Fig. 15 in einer Fixierstellung, in der der Hebel 50 umgelegt ist und der Schwenkkörper 48 unter Fixierung des Führungsdrahts 51 gegen Längsverschiebungen an die Gegenplatte 47 angedrückt ist. In der in Fig. 16 dargestellten Fixierstellung ist der Hebel 50 mit in Fig. 16 nicht dargestellten Arretiermitteln fixierbar.

## Patentansprüche

1. Vorrichtung zur Handhabung wenigstens eines Führungsdrahts (51) zur Führung eines interventionellen medizinischen Instruments oder zur Handhabung eines Katheterschafts bei interventionellen medizinischen Techniken, **gekennzeichnet durch** einen an einem Einführventil (1) befestigbaren Fixierarm (13) und eine an dem Fixierarm (13) angebrachten Klemmvorrichtung (16, 38, 46), mit der der betreffende Führungsdraht (51) beziehungsweise Katheterschaft gegen Längsverschiebung gesichert fixierbar ist.

2. Vorrichtung nach Anspruch 1, **dadurch gekennzeichnet, daß** der Fixierarm (13) über eine Nut-Feder-Verbindung (19, 20) lösbar an dem Einführventil (1) befestigbar ist.

3. Vorrichtung nach Anspruch 1, **dadurch gekennzeichnet, daß** der Fixierarm (13) über eine Steckverbindung lösbar an dem Einführventil (1) befestigbar ist.

4. Vorrichtung nach Anspruch 2, **dadurch gekennzeichnet, daß** ein Adapterstück vorgesehen ist, mit dem der Fixierarm (13) an dem Einführventil (1) lösbar befestigbar ist.

5. Vorrichtung nach Anspruch 1, **dadurch gekennzeichnet, daß** der Fixierarm (13) fest mit dem Einführventil (1) verbunden ist.

6. Vorrichtung nach einem der Ansprüche 1 bis 5,**dadurch gekennzeichnet, daß** der Fixierarm (13) über ein Einführende (7) des Einführventils (1) vorsteht.

7. Vorrichtung nach Anspruch 6, **dadurch gekennzeichnet, daß** der Fixierarm (13) einen Befestigungsabschnitt (14) und einen gegen den Befestigungsabschnitt (14) abgewinkelten Halteabschnitt (15) aufweist.

8. Vorrichtung nach Anspruch 6, **dadurch gekennzeichnet, daß** der Fixierarm (13) einen Befestigungsabschnitt (14) und einen als Halteplatte (17) ausgebildeten Halteabschnitt aufweist.

9. Vorrichtung nach einem der Ansprüche 1 bis 8, **dadurch gekennzeichnet, daß** die Klemmvorrichtung eine Klemmeinheit (16, 38, 46) zur Fixierung eines Führungsdrahts (51) aufweist.

10. Vorrichtung nach einem der Ansprüche 1 bis 8, **dadurch gekennzeichnet, daß** die Klemmvorrichtung wenigstens zwei jeweils in einem seitlichen Abstand angeordnete Klemmeinheiten (16) zur Fixierung von zwei Führungsdrähten (51) oder einem Führungsdraht (51) und einem Katheterschaft aufweist.

11. Vorrichtung nach einem der Ansprüche 1 bis 8, **dadurch gekennzeichnet, daß** die Klemmvorrichtung drei oder vier jeweils in einem seitlichen Abstand angeordnete Klemmeinheiten (16) zur Fixierung von Führungsdrähten (51) und/oder Katheterschäften aufweist.

12. Vorrichtung nach einem der Ansprüche 1 bis 11, **dadurch gekennzeichnet, daß** die Klemmvorrichtung als Klemmeinheit wenigstens eine längliche Klemmschiene (16) aufweist, die über ein Fassungsteil mit einer Grundplatte (22) sowie zwei Seitenwangen (23, 24, 29, 30) und über einen in das Fassungsteil eingefügten, mit einem längs verlaufenden Klemmschlitz (26) ausgebildeten Klemmkörper (25) verfügt.

13. Vorrichtung nach Anspruch 12, **dadurch gekennzeichnet, daß** die Grundplatte (22) sowie die Seitenwangen (23, 24) fest miteinander verbunden sind und rechtwinklig zueinander stehen.

14. Vorrichtung nach Anspruch 12, **dadurch gekennzeichnet, daß** die Seitenwangen (29, 30) jeweils über ein Scharnier (31, 32) schwenkbar an der Grundplatte (22) angebracht sind und daß eine Verriegelungsvorrichtung (33, 34; 35, 36, 37) zur Fixierung der Seitenwangen (29, 30) bei geschlossenem Klemmschlitz (26) vorgesehen ist.

15. Vorrichtung nach Anspruch 14, **dadurch gekennzeichnet, daß** die Verriegelungsvorrichtung zwei Schnappnasen (33, 34) aufweist, die sich in geschlossener Stellung der Seitenwangen (29, 30) gegenseitig hintergreifen.

16. Vorrichtung nach Anspruch 14, **dadurch gekennzeichnet, daß** die Verriegelungsvorrichtung einen Kippbügel (35) aufweist, der mit einem Ende gelenkig an einer Seitenwange (29, 30) angebracht ist und in geschlossener Stellung die andere Seitenwange (30, 29) übergreift.

17. Vorrichtung nach einem in der Ansprüche 12 bis 16, **dadurch gekennzeichnet, daß** der Klemmschlitz (26) gewellt ist.

18. Vorrichtung nach einem der Ansprüche 12 bis 17, **dadurch gekennzeichnet, daß** der Klemmkörper (25) zu dem Klemmschlitz (26) quer oder schräg ausgerichtete Schlitze (27, 28) aufweist.

19. Vorrichtung nach einem der Ansprüche 1 bis 11, daß die Klemmvorrichtung wenigstens eine Klemmhülsenanordnung (38) mit einer Klemmhülse (39) und einer Schraubhülse (40) aufweist, die mit zueinander ausrichtbaren, zur Aufnahme eines Führungsdrahts (51) oder eines Katheterschafts vorgesehene Nuten ausgebildet sind, wobei sich in einer Drehrichtung der Querschnitt der Klemmhülse (39) wenigstens abschnittsweise verringert.

20. Vorrichtung nach einem der Ansprüche 1 bis 11, **dadurch gekennzeichnet, daß** die Klemmvorrichtung eine Gegenplatte (47) und einen drehbar gelagerten Schwenkkörper (48) aufweist, der in einer ersten Schwenkstellung einen Abstand von der Gegenplatte (47) aufweist und in einer zweiten Schwenkstellung abschnittsweise an der Gegenplatte (47) anliegt.

21. Vorrichtung nach Anspruch 20, **dadurch gekennzeichnet, daß** der Schwenkkörper einen kreisartigen Querschnitt aufweist und azentrisch gelagert ist.

22. Vorrichtung nach Anspruch 20, **dadurch gekennzeichnet, daß** der Schwenkkörper (48) einen ellipsenartigen Querschnitt aufweist.

23. Vorrichtung nach Anspruch 22, **dadurch gekennzeichnet, daß** der Schwenkkörper (48) mittig gelagert ist.

24. Vorrichtung nach Anspruch 22, **dadurch gekennzeichnet, daß** der Schwenkkörper (48) im Bereich eines Brennpunkts gelagert ist.

## Claims

1. Device for handling at least one guide wire (51) for guiding an invasive medical instrument or for handling a catheter shaft in invasive medical techniques, **characterized by** a fixing arm (13) which can be secured on an introduction valve (1), and a clamping device (16, 38, 46) which is arranged on the fixing arm (13) and with which the particular guide wire or catheter shaft can be secured against longitudinal displacement.

2. Device according to claim 1, **characterized in that** the fixing arm (13) can be secured on the introduction valve (1) in a releasable manner via a groove-and-tongue connection.

3. Device according to claim 1, **characterized in that** the fixing arm (13) can be secured on the introduction valve (1) in a releasable manner via a plug connection.

4. Device according to claim 2, **characterized in that** an adapter piece is provided with which the fixing arm (13) can be secured in a releasable manner on the introduction valve (1).

5. Device according to claim 1, **characterized in that** the fixing arm (13) is connected rigidly to the introduction valve (1).

6. Device according to any of claims 1 through 5, **characterized in that** the fixing arm (13) protrudes beyond an introduction end (7) of the introduction valve (1).

7. Device according to claim 6, **characterized in that** the fixing arm (13) comprises a securing portion (14) and a holding portion (15) which is angled off in relation to the securing portion.

8. Device according to claim 6, **characterized in that** the fixing arm (13) comprises a securing portion (14) and a holding portion (17) designed as a holding plate (17).

9. Device according to any of claims 1 through 8, **characterized in that** the clamping device has a clamping unit (16, 38, 46) for fixing a guide wire (51).

10. Device according to any of claims 1 through 8, **characterized in that** the clamping device has at least two clamping units (16) which are arranged laterally spaced apart from one another and are used for fixing two guide wires (51) or one guide wire (51) and a catheter shaft.

11. Device according to any of claims 1 through 8, **characterized in that** the clamping device has three or four clamping units (16) which are arranged laterally spaced apart from one another and are used for fixing guide wires (51) and/or catheter shafts.

12. Device according to any of claims 1 through 11, **characterized in that** the clamping device has as clamping unit at least one elongated clamping rail (16) which has a holder part with a base plate (22) and two side cheeks (23, 24, 29, 30) and has a clamping body (25) inserted into the holder part and designed with a longitudinally extending clamping slit (26).

13. Device according to claim 12, **characterized in that** the base plate (22) and the side cheeks (23, 24) are connected rigidly to one another and at right angles to one another.

14. Device according to claim 12, **characterized in that** the side cheeks (29, 30) are each arranged pivotably on the base plate (22) by a respective hinge (31, 32), and **in that** a locking device (33, 34; 35, 36, 37) is provided for fixing the side cheeks (29, 30) with the clamping slit (26) closed.

15. Device according to claim 14, **characterized in that** the locking device has tow snap-fit lugs (33, 34) which engage one behind the other in the closed position of the side cheeks (29, 30).

16. Device according to claim 14, **characterized in that** the locking device has a tilting bracket (35) which at one end is articulated on one side cheek (29, 30) and in the closed position engages over the other side cheek (30, 29).

17. Device according to claim 12, **characterized in that** the clamping slit is corrugated.

18. Device according to any of claims 12 through 17, **characterized in that** the clamping body (25) has slits (27, 28) which are oriented transversely or obliquely with respect to the clamping slit (26).

19. Device according to any of claims 1 through 11, **characterized in that** the clamping device has at least one clamping sleeve arrangement (38) with a clamping sleeve (39) and a screw sleeve (40) which are designed with grooves that can be aligned relative to one another and are intended to receive a guide wire (51) or a catheter shaft, the cross section of the clamping sleeve (39) decreasing at least in stages in a direction of rotation.

20. Device according to any of claims 1 through 11, **characterized in that** the clamping device has a counter plate (47) and a rotatably mounted pivot body (48) which, in a first pivot position, is at a distance from the counter plate (47), and, in a second pivot position, lays in part on the counter plate (47).

21. Device according to claim 20, **characterized in that** the pivot body (48) has a circular cross section and is mounted eccentrically.

22. Device according to claim 20, **characterized in that** the pivot body (48) has an elliptical cross section.

23. Device according to claim 22, **characterized in that** the pivot body (48) is mounted centrally.

24. Device according to claim 22, **characterized in that** the pivot body (48) is mounted in the area of a focal point.

## Revendications

1. Dispositif pour manipuler au moins un fil de guidage (51) destiné à guider un instrument médical d'intervention ou pour manipuler une tige de cathéter lors de techniques médicales d'intervention, **caractérisé par** un bras de fixation (13) pouvant être fixé à une soupape d'introduction (1), et par un dispositif de serrage (16, 38, 46) placé sur le bras de fixation (13), au moyen duquel le fil de guidage (51) concerné ou la tige de cathéter peut être fixé de manière à ne pouvoir coulisser longitudinalement.

2. Dispositif selon la revendication 1, **caractérisé en ce que** le bras de fixation (13) peut être fixé par une liaison à rainure et languette (19, 20), de manière séparable, à la soupape d'introduction (1).

3. Dispositif selon la revendication 1, **caractérisé en ce que** le bras de fixation (13) peut être fixé de manière séparable, par une liaison à enfichage, à la soupape d'introduction (1).

4. Dispositif selon la revendication 2, **caractérisé en ce qu'**il est prévu un adaptateur au moyen duquel le bras de fixation (13) peut être fixé de manière séparable à la soupape d'introduction (1).

5. Dispositif selon la revendication 1, **caractérisé en ce que** le bras de fixation (13) est relié fixement à la soupape d'introduction (1).

6. Dispositif selon l'une des revendications 1 à 5, caractérisé en ce le bras de fixation (13) dépasse d'une extrémité d'introduction (7) de la soupape d'introduction (1).

7. Dispositif selon la revendication 6, **caractérisé en ce que** le bras de fixation (13) comporte un tronçon de fixation (14) et un tronçon de maintien (15) coudé vers le tronçon de fixation (14).

8. Dispositif selon la revendication 6, **caractérisé en ce que** le bras de fixation (13) comporte un tronçon de fixation (14) et un tronçon de maintien réalisé en tant que plaque de maintien (17).

9. Dispositif selon l'une des revendications 1 à 8, **caractérisé en ce que** le dispositif de serrage comporte une unité de serrage (16, 38, 46) pour fixer un fil de guidage (51).

10. Dispositif selon l'une des revendications 1 à 8, **caractérisé en ce que** le dispositif de serrage comporte au moins deux unités de serrage (16), disposées à distance latérale, pour fixer deux fils de guidage (51) ou un fil de guidage (51) et une tige de cathéter.

11. Dispositif selon l'une des revendications 1 à 8, **caractérisé en ce que** le dispositif de serrage comporte trois ou quatre unités de serrage (16) disposées à distance latérale, pour fixer des fils de guidage (51) et/ou des tiges de cathéter.

12. Dispositif selon l'une des revendications 1 à 11, **caractérisé en ce que** le dispositif de serrage comporte en tant qu'unité de serrage au moins un rail de serrage (16) allongé qui comporte une monture avec une plaque de base (22) ainsi que deux joues latérales (23, 24, 29, 30) et un corps de serrage (25) inséré dans la monture, et présentant une fente de serrage (26) s'étendant longitudinalement.

13. Dispositif selon la revendication 12, **caractérisé en ce que** la plaque de base (22) ainsi que les joues latérales (23, 24) sont reliées fixement entre elles et sont perpendiculaires les unes aux autres.

14. Dispositif selon la revendication 12, **caractérisé en ce que** les joues latérales (29, 30) sont placées sur la plaque de base de manière à pouvoir pivoter chacune au moyen d'une charnière (31, 32), et **en ce qu'**un dispositif de verrouillage (33, 34 ; 35, 36, 37) est prévu pour fixer les joues latérales (29, 30) lorsque la fente de serrage (26) est fermée.

15. Dispositif selon la revendication 14, **caractérisé en ce que** le dispositif de verrouillage comporte deux ergots à déclic (33, 34) qui, en position fermée des joues latérales (29, 30), passent réciproquement l'un derrière l'autre.

16. Dispositif selon la revendication 14, **caractérisé en ce que** le dispositif de verrouillage comporte un étrier basculant (36) qui, par une extrémité, est placé de manière articulée sur une joue latérale (29, 30) et qui, en position fermée, passe sur l'autre joue latérale (30, 29).

17. Dispositif selon l'une des revendications 12 à 16, **caractérisé en ce que** la fente de serrage (26) est ondulée.

18. Dispositif selon l'une des revendications 12 à 17, **caractérisé en ce que** le corps de serrage (25) comporte des fentes (27, 28) orientées transversalement ou obliquement par rapport à la fente de serrage (26).

19. Dispositif selon l'une des revendications 1 à 11, **caractérisé en ce que** le dispositif de serrage comporte au moins un agencement de douille de serrage (38) avec une douille de serrage (39) et une douille de vissage (40) qui présentent des rainures pouvant être orientées l'une par rapport à l'autre, prévues pour recevoir un fil de guidage (51) ou une tige de cathéter, la section transversale de la douille de serrage (39) se rétrécissant au moins par endroits dans un sens de rotation.

20. Dispositif selon l'une des revendications 1 à 11, **caractérisé en ce que** le dispositif de serrage comporte une contre-plaque (47) et un corps pivotant (48) monté tournant qui, dans une première position de pivotement, présente une distance par rapport à la contre-plaque (47) et qui, dans une deuxième position de pivotement, s'applique par endroits contre la contre-plaque (47).

21. Dispositif selon la revendication 20, **caractérisé en ce que** le corps pivotant (48) présente une section transversale de type circulaire et est monté non centré.

22. Dispositif selon la revendication 20, **caractérisé en ce que** le corps pivotant (48) présente une section transversale de type ellipse.

23. Dispositif selon la revendication 22, **caractérisé en ce que** le corps pivotant (48) est monté au centre.

24. Dispositif selon la revendication 22, **caractérisé en ce que** le corps pivotant (48) est monté dans la zone d'un foyer.
